# EUROPEAN PATENT APPLICATION

(11) **EP 4 696 330 A1**
(43) Date of publication of application: **18.02.2026**
(21) Application number: 24788181.6
(22) Date of filing: 12.04.2024
(51) Int. Cl.: A61K 47/68, A61K 39/00, C07K 16/18, A61K 39/395, A61P 35/00

(54) **USE OF ANTIBODY-DRUG CONJUGATE IN PREPARATION OF DRUG FOR PREVENTING AND/OR TREATING CANCER**

(30) Priority: 12.04.2023 CN 202310390337
(71) Applicant: Shanghai Hansoh Biomedical Co., Ltd., Shanghai 201203 (CN); Changzhou Hansoh Pharmaceutical Co., Ltd., Xinbei District Changzhou Jiangsu 213001 (CN)
(72) Inventor: SUN, Danni, Shanghai 201203 (CN); LIU, Junhao, Shanghai 201203 (CN); ZHOU, Yuanfeng, Shanghai 201203 (CN); HAN, Yang, Shanghai 201203 (CN)
(74) Representative: Wilkinson, Johanna Elise
(86) International application number: PCT/CN2024/087385
(87) International publication number: WO 2024/213081

(57) **Abstract**

The present invention relates to the use of an antibody-drug conjugate or its pharmaceutically acceptable salts, stereoisomers, metabolites or solvent compounds in preparation of drugs for prevention and/or treatment of cancers. Specifically, the present invention provides the use of an anti-B7-H4 antibody-drug conjugate or its pharmaceutically acceptable salts, stereoisomers, metabolites or solvent compounds in preparation of drugs for prevention and/or treatment of cancers, especially cholangiocarcinomas with low B7-H4 expression. The present invention also provides a drug formulation comprising B7-H4 antibody-drug conjugate or its pharmaceutically acceptable salts, stereoisomers, metabolites or solvent compounds.

## Description

### Technical Field

The present application belongs to the medical field and relates to the use of an antibody-drug conjugate in preparation of drugs for prevention and/or treatment of cancer diseases.

### Background Art

Cholangiocarcinoma originates from biliary epithelial cells, accounting for about 3% of gastrointestinal tumours and 10%-15% of hepatobiliary malignancies. Cholangiocarcinoma shows higher incidence in Asian countries than in European and American countries, affecting 113/100,000 men and 50/100,000 women. Radical operation is the only cure for cholangiocarcinoma. However, the disease presents with no characteristic clinical manifestations at the early stage and has been terminal when identified in the overwhelming majority of patients, resulting in very poor prognosis with an about 10% 5-year survival rate. Up to 50% of cholangiocarcinoma recurs one year even after surgical excision. In unresectable locally progressive and metastatic bile duct tumours (including intrahepatic and extrahepatic cholangiocarcinoma, gallbladder carcinoma and ampullary carcinoma), the standard first-line chemotherapy regimen [gemcitabine plus cis-platinum] only brings a median survival period of 11.7 months, indicating very poor prognosis. Drugs targeting BRAF, IDH1, FGFR2, HER2 and PD-1/PD-L1 have been approved for treatment of advanced biliary malignancies, but their therapeutic effects are limited and new therapies are urgently needed to improve the limitations of treatment of patients with advanced cholangiocarcinoma.

Antibody-drug conjugates (ADCs) are a class of drugs that play their roles by chemically bonding bioactive cytotoxic drugs to monoclonal antibodies, which transport cytotoxic drugs to the target cells as carriers. ADCs are mainly used in the tumour field at present and generally require high expression of antigen targets in tumour cells. B7-H4 is a new member of the newly discovered B7 family that plays an important role in several biological processes of cells such as differentiation, proliferation and apoptosis and may impact the invasion and metastasis of tumour cells. The B7 family are also important costimulatory molecules that may impact T cell proliferation, B cell activation and other processes. A study has shown high B7-H4 expression in many tumours including cholangiocarcinoma, breast cancer, endometrial cancer, non-small cell lung cancer, ovarian cancer, gastric cancer, and pancreatic cancer and limited expression in normal tissues, so B7-H4 is expected to become a target of ADCs.

B7-H4 is highly expressed in cholangiocarcinoma and patients with high B7-H4 expression have poorer prognosis, indicating that B7-H4 can be used as a potential target for treatment of cholangiocarcinoma. It has been reported that antibodies or ADCs targeting B7-H4 can be used for cancers with high B7-H4 expression including cholangiocarcinoma, but the therapeutic effects of these drugs in cholangiocarcinoma with low B7-H4 expression are unknown.

### Summary of the Invention

The first aspect of the present invention provides the use of an anti-B7-H4 antibody-drug conjugate or its pharmaceutically acceptable salts, stereoisomers, metabolites or solvent compounds in preparation of drugs for prevention and/or treatment of cancers, wherein the anti-B7-H4 antibody-drug conjugate has the structure represented by formula (I):

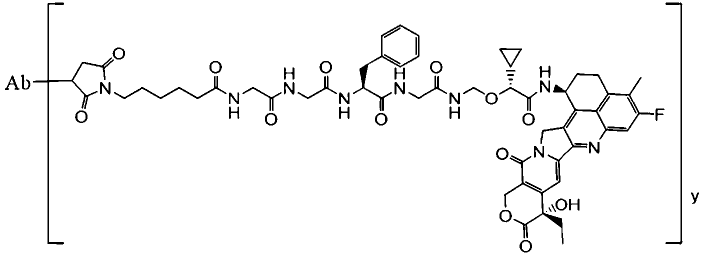

Wherein y represents 1 to 20, preferably, y represents 2 to 8, and more preferably, y represents 4 to 6 or 7 to 8; y is a decimal or integer, and further preferably, y represents 5.6, 5.7, 5.8, 5.9, 6.0, 6.1, 6.2, 6.3, or 6.4;

Ab is an anti-B7-H4 antibody or its antigen-binding fragment thereof, comprising a variable region of antibody heavy chain and a variable region of antibody light chain, wherein the variable region of antibody heavy chain comprises HCDR1, HCDR2 and HCDR3 as shown in SEQ ID NO:1, SEQ ID NO:2 and SEQ ID NO:3 respectively; the variable region of antibody light chain comprises LCDR1, LCDR2 and LCDR3 as shown in SEQ ID NO:4, SEQ ID NO:5 and SEQ ID NO:6 respectively; and the said cancers are those with low B7-H4 expression.

In some embodiments, the anti-B7-H4 antibody or its antigen-binding fragment thereof is a humanised antibody or its fragment.

In some embodiments, the humanised antibody comprises a variable region of heavy chain as shown in SEQ ID NO:7 and a variable region of light chain as shown in SEQ ID NO:8; preferably, the humanised antibody comprises a heavy chain as shown in SEQ ID NO:9 and a light chain as shown in SEQ ID NO: 10.

In some embodiments, the cancers with low B7-H4 expression are characterised by a B7-H4 expression level of IHC 1+ or 2+.

In some embodiments, the cancers with low B7-H4 expression are cases with IHC 1+ or 2+ B7-H4 expression in at least 1% of tumour cells numerically or IHC 1+ or 2+ B7-H4 expression in 0-1% of cells numerically by IHC in samples from clinical patients.

In some embodiments, the cancers with low B7-H4 expression are cholangiocarcinoma with low B7-H4 expression, preferably cholangiocarcinoma with an IHC 1+ or IHC 2+ B7-H4 expression level.

In some embodiments, the cancers with low B7-H4 expression are cholangiocarcinoma with low B7-H4 expression, preferably cholangiocarcinoma with an IHC 1+ or IHC 2+ B7-H4 expression level in clinical testing.

In some embodiments, the prevention and/or treatment includes administration of a therapeutically effective dose of the anti-B7-H4 antibody-drug conjugate in formula (I) or its pharmaceutically acceptable salts, stereoisomers, metabolites or solvent compounds to patients at 0.5-15 mg/kg of body weight, and preferably at 3-10 mg/kg of body weight.

In some embodiments, the prevention and/or treatment includes administration of a therapeutically effective dose of the anti-B7-H4 antibody-drug conjugate in formula (I) or its pharmaceutically acceptable salts, stereoisomers, metabolites or solvent compounds to patients via oral, parenteral, or transdermal route, wherein the parenteral route is selected from intravenous, subcutaneous or intramuscular injection.

In some embodiments, the prevention and/or treatment includes administration of a therapeutically effective dose of the anti-B7-H4 antibody-drug conjugate in formula (I) or its pharmaceutically acceptable salts, stereoisomers, metabolites or solvent compounds to patients, wherein the drug is an injectable form, for example, subcutaneous or intravenous injection; the preferable injectable form is injection or lyophilised powder for injection, comprising the anti-B7-H4 antibody-drug conjugate in formula (I) or its pharmaceutically acceptable salts, stereoisomers, metabolites or solvent compounds, and buffers, stabilisers, pH regulators and optional existing surfactants.

The second aspect of the present invention provides a method for prevention and/or treatment of cancers with low B7-H4 expression, wherein the method includes administration of a therapeutically effective dose of the anti-B7-H4 antibody-drug conjugate in formula (I) as defined above or its pharmaceutically acceptable salts, stereoisomers, metabolites or solvent compounds to those in need, preferably at 0.5-15 mg/kg of body weight, and more preferably at 3-10 mg/kg of body weight;
or, the method includes administration of a drug combination with a therapeutically effective dose of the anti-B7-H4 antibody-drug conjugate in formula (I) as defined above or its pharmaceutically acceptable salts, stereoisomers, metabolites or solvent compounds to those in need;

The cancers with low B7-H4 expression are those with an IHC 1+ or 2+ B7-H4 expression level; preferably, the cancers with low B7-H4 expression are bile duct cancers with low B7-H4 expression; and more preferably, the cancers with low B7-H4 expression are bile duct cancers with an IHC 1+ or IHC 2+ B7-H4 expression level.

In some embodiments, the cancers with low B7-H4 expression are cases with IHC 1+ or 2+ B7-H4 expression in at least 1% of tumour cells numerically or IHC 1+ or 2+ B7-H4 expression in 0-1% of cells numerically by IHC in samples from clinical patients; preferably, the cancers with low B7-H4 expression are cholangiocarcinoma with low B7-H4 expression; and more preferably, the cancers with low B7-H4 expression are cholangiocarcinoma with an IHC 1+ or IHC 2+ B7-H4 expression level in clinical testing.

In some embodiments, the drug in the aforementioned prevention and/or treatment method is administered via oral, parenteral, or transdermal route, wherein the parenteral route is selected from intravenous, subcutaneous or intramuscular injection;

In some embodiments, the drug in the aforementioned prevention and/or treatment includes is an injectable form, for example, subcutaneous or intravenous injection; a more preferable injectable form is injection or lyophilised powder for injection, comprising the anti-B7-H4 antibody-drug conjugate in formula (I) or its pharmaceutically acceptable salts, stereoisomers, metabolites or solvent compounds, and buffers, stabilisers, pH regulators and optional existing surfactants.

The third aspect of the present invention provides a drug formulation including a therapeutically effective dose of the anti-B7-H4 antibody-drug conjugate in formula (I) as defined above or its pharmaceutically acceptable salts or metabolites or solvent compounds, which also includes a pharmaceutically acceptable carrier, wherein the anti-B7-H4 antibody-drug conjugate is administered at 0.5-15 mg/kg of body weight, preferably at 3-10 mg/kg of body weight; the drug formulation is used to treat cancers with low B7-H4 expression, wherein the cancers with low B7-H4 expression are those with an IHC 1+ or 2+ B7-H4 expression level; preferably, the cancers with low B7-H4 expression are bile duct cancers with low B7-H4 expression; and more preferably, the cancers with low B7-H4 expression are bile duct cancers with an IHC 1+ or IHC 2+ B7-H4 expression level.

In some embodiments, the cancers with low B7-H4 expression are cases with IHC 1+ or 2+ B7-H4 expression in at least 1% of tumour cells numerically or IHC 1+ or 2+ B7-H4 expression in 0-1% of cells numerically by IHC in samples from clinical patients; preferably, the cancers with low B7-H4 expression are cholangiocarcinoma with low B7-H4 expression; and more preferably, the cancers with low B7-H4 expression are cholangiocarcinoma with an IHC 1+ or IHC 2+ B7-H4 expression level in clinical testing.

The anti-B7-H4 antibody-drug conjugate of the present invention significantly inhibits the growth of xenografts of human cholangiocarcinoma LD1-0060-202214 with low B7-H4 expression in nude mice and does not cause significant weight loss or other abnormal symptoms in the mice, showing an excellent prospect of clinical use.

### Brief Description of the Figures

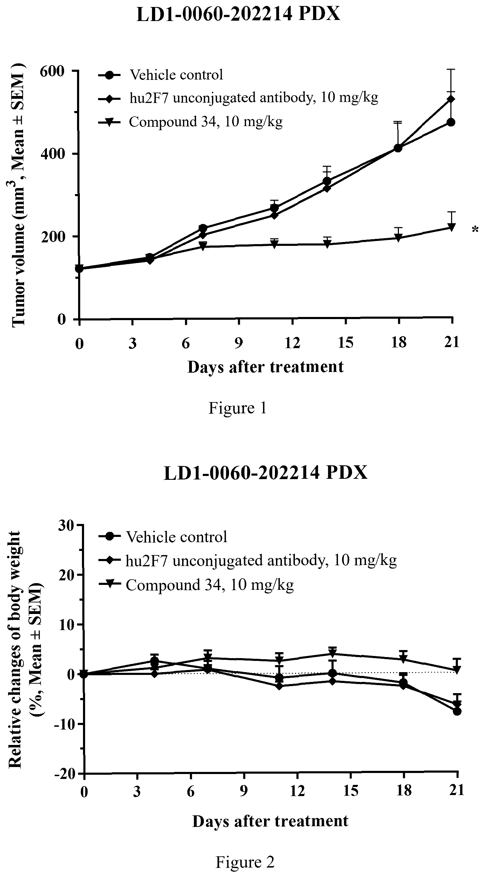

Figure 1: Efficacy of Anti-B7-H4 Antibody-Drug Conjugate in Subcutaneous Xenografts of Human Cholangiocarcinoma LD1-0060-202214 in Mice
Figure 2: Effects of Anti-B7-H4 Antibody-Drug Conjugate on Weight of Mice with Human Cholangiocarcinoma LD1-0060-202214

### Detailed Description of Embodiments

### I. Terms

In order to better understand the present invention, some technical and scientific terms are defined below in detail. Unless obviously defined otherwise in the document, all technical and scientific terms used herein have the meanings generally understandable to those skilled in the art of the present invention.

The three-letter codes and single-letter codes for amino acids used in the present invention are described in J. Biol. Chem, 243, p3558(1968).

The term "antibody" as used in the present invention refers to an immunoglobulin, a tetrameric structure composed of two identical heavy chains and two identical light chains, which are connected via interchain disulfide bonds. The amino acid composition and sequence of the constant region of the heavy chain differ among immunoglobulins, resulting in distinct antigenic properties. Accordingly, immunoglobulins can be classified into five major classes, also known as isotypes: IgM, IgD, IgG, IgA, and IgE, corresponding to the µ, δ, γ, α, and ε heavy chains, respectively. Each immunoglobulin class can be further subdivided into subclasses based on differences in the amino acid composition of the hinge region and the number and position of disulfide bonds in the heavy chain. For example, IgG can be subdivided into IgG1, IgG2, IgG3, and IgG4. Light chains are classified as κ (kappa) or λ (lambda) based on their constant regions. Each of the five immunoglobulin classes may contain either κ or λ light chains.

In the present invention, the variable region of the light chain may further include a constant region. The constant region of the light chain comprises a human or murine κ or λ chain, or a variant thereof.

Similarly, the variable region of the heavy chain may further include a constant region. The constant region of the heavy chain comprises human or murine IgG1, IgG2, IgG3, or IgG4, or variants thereof.

The N-terminal region (approximately the first 110 amino acids) of both the heavy and light chains shows considerable sequence variability and is referred to as the variable region (V region). The remaining C-terminal region has relatively conserved amino acid sequences and is known as the constant region (C region). Each variable region consists of three hypervariable regions (HVRs) and four relatively conserved framework regions (FRs). The three hypervariable regions determine the antigen-binding specificity of the antibody and are also referred to as complementarity-determining regions (CDRs). The variable regions of the light chain (VL) and the heavy chain (VH) each consist of three CDRs and four FRs, arranged sequentially from the N-terminus to the C-terminus as follows: FR1, CDR1, FR2, CDR2, FR3, CDR3 and FR4. The three CDRs of the light chain are referred to as LCDR1, LCDR2, and LCDR3, and the three CDRs of the heavy chain are referred to as HCDR1, HCDR2, and HCDR3, respectively. The CDR amino acid residues in the VL and VH regions of the antibody or antigen-binding fragment of the present invention conform to the known Kabat numbering rules and the definitions of Kabat or AbM with respect to their number and position.

The term "antigen-presenting cell" or "APC" refers to a cell that displays foreign antigens on its surface in association with major histocompatibility complex (MHC) molecules. T cells recognise these complexes via their T cell receptors (TCRs). Examples of APCs include, but are not limited to, dendritic cells (DCs), peripheral blood mononuclear cells (PBMCs), monocytes, B lymphoblasts, and monocyte-derived dendritic cells (DCs). The term "antigen presentation" refers to the process by which APCs capture antigens and present them to T cells for recognition, for example as part of MHC class I or class II complexes.

The term "B7-H4" refers to a member of the human B7 protein family, also known as CD276, which is a type I transmembrane protein containing four immunoglobulin-like extracellular domains. B7-H4 is one of the immune checkpoint proteins expressed on the surface of antigen-presenting cells or cancer cells and is known to inhibit T cell activation. The term "B7-H4" also encompasses any variants or isotypes of B7-H4 that are naturally expressed in cells. The antibody of the present invention may cross-react with B7-H4 derived from non-human species. Alternatively, the antibody may be specific to human B7-H4 and may exhibit no cross-reactivity with B7-H4 from other species. B7-H4, or any of its variants or isotypes, may be isolated from cells or tissues that naturally express it, or may be produced recombinantly using techniques known in the art and as described herein. Preferably, anti-B7-H4 antibodies target human B7-H4 with normal glycosylation pattern.

The term "humanised antibody", also referred to as a CDR-grafted antibody, refers to an antibody in which CDRs derived from a mouse antibody are grafted into the framework regions of a human antibody variable domain. This overcomes the strong immune responses induced by the massive mouse protein elements carried in chimeric antibodies. To minimise the loss of activity associated with reduced immunogenicity, minimal back mutations may be introduced into the said human antibody variable region to retain binding activity.

The term "antigen-binding fragment" refers to antigen-binding fragments of antibody and antibody analogues and usually includes the antigen-binding regions or variable regions (such as one or more CDRs) of at least partial parental antibodies. The antibody fragment retains at least a portion of the binding specificity of the parental antibody. In general, when binding activity is measured on a molar basis, the antibody fragment retains at least 10% of the binding activity of the parental antibody. Preferably, the antibody fragment retains at least 20%, 50%, 70%, 80%, 90%, 95%, or even 100% or more of the binding affinity of the parental antibody for the target antigen. Examples of antigen-binding fragment include but are not limited to: Fab, Fab', F(ab')2, Fv fragments, linear antibodies, single-chain antibodies, nanobodies, domain antibodies and multispecific antibodies. The engineered antibody variants are summarised in Holliger and Hudson (2005) Nat. Biotechnol. 23:1126-1136.

The term "drug combination" refers to a product comprising one or more active ingredients (e. g., antibody, antibody-drug conjugate, small molecule drug) in optionally defined amounts, and any product resulting, directly or indirectly, from combining one or more active ingredients, also in optionally defined amounts. These combinations can also optionally comprise appropriate pharmaceutical adjuvants, such as pharmaceutical carriers and pharmaceutical excipients known in the art, including buffers. The different active ingredients in a drug combination can be administered independently in separate dosage forms, either at the same or different time points for a synergistic effect. In the present disclosure, "drug combination" and "drug formulation" are not mutually exclusive.

The term "pharmaceutically acceptable salts" refers to salts of the antibody-drug conjugate that are safe, effective, and retain appropriate bioactivity when administered to a mammal. The antibody-drug conjugate in the present invention contains at least one amino and therefore can form a salt with acid. Unrestricted examples of pharmaceutically acceptable salts include: hydrochloride, hydrobromide, hydriodate, sulphate, disulphate, citrate, acetate, succinate, ascorbate, oxalate, nitrate, sorbate, hydrophosphate, dihydrogenphosphate, salicylate, sodium citrate monobasic, tartrate, maleate, fumarate, formate, benzoate, mesylate, esilate, benzenesulphonate, and tosylate."Pharmaceutically acceptable salts" and "pharmaceutical salts" can be used interchangeably.

"Solvent compounds" refer to a pharmaceutical solvent compound of the antibody-drug conjugate in the present invention and one or more solvent molecules. Unrestricted examples of solvent molecules include: water, ethanol, acetonitrile, isopropanol, and ethyl acetate.

"Drug-to-antibody ratio" (DAR) is described as y, i. e., the mean quantity of cytotoxic drugs per antibody in formula (I). the DAR may range from 1 to 20 cytotoxic drugs (D) per antibody molecule. The antibody-drug conjugates represented by general formula (A) constitute a group of antibodies conjugated with a defined number (1-20) of cytotoxic drug molecules. The DAR of the antibody-drug conjugates resulting from the conjugation reaction can be determined by conventional analytical techniques, including mass spectrometry, high-performance liquid chromatography (HPLC), and enzyme-linked immunosorbent assay (ELISA). The quantitative distribution of the antibody-drug conjugate in y value can be measured by these analytical methods.

The term "low B7-H4 expression" generally refers to a B7-H4 expression level of IHC 1+ or 2+, as detected by immunohistochemistry (IHC) in at least 1% of tumour cells in clinical samples. Cases in which 0-1% of tumour cells exhibit B7-H4 expression at IHC 1+ or 2+ are also considered within the scope of "low B7-H4 expression" in the present invention. The terms "high B7-H4 expression" and "B7-H4 overexpression" are used interchangeably and generally refer to IHC 3+ expression as determined in clinical testing.

The following embodiments are provided to further illustrate the present invention. However, these embodiments are not intended to limit the scope of the invention in any way. Unless otherwise specified, the experimental methods described in the embodiments of the present invention were performed under standard conditions, such as those described in the antibody technical experiment manual and molecular cloning manual from The Cold Spring Harbor Laboratory, or according to the instructions provided by the manufacturer of the raw materials or reagents. Reagents without specified sources are commercially available standard reagents.

### II. Biological Assessment Method

### Embodiment 1. In Vivo Pharmacodynamic Study of Anti-B7-H4 Antibody-Drug Conjugate in the Xenograft Model of Human Cholangiocarcinoma LD1-0060-202214 in NU/NU Mice

### 1. Experimental Objective

To assess the in vivo pharmacodynamics of anti-B7-H4 antibody-drug conjugate in the xenograft model of human cholangiocarcinoma LD1-0060-202214 in NU/NU mice.

### 2. Experimental Materials

### 2.1 Information of human cholangiocarcinoma LD1-0060-202214 PDX model

LD1-0060-202214, a human cholangiocarcinoma tumour tissue, has been passaged to generation FP3+3 for this pharmacodynamic test and is provided by Shanghai LIDE Biotech Co., Ltd.

**Table 1. Model Information**

| **Model No.** | **Cancer Type** | **Pathological Diagnosis** | **B7-H4 Expression** |
|---|---|---|---|
| LD 1-0060-202214 | Cholangiocarcinoma | Poorly to moderately differentiated adenocarcinoma | Low expression (IHC1+) |

### 2.2 Investigational drug

1) Anti-B7-H4 antibody (hu2F7 unconjugated antibody, Shanghai Hansoh BioMedical Co., Ltd., batch number: DS202207): the anti-B7-H4 antibody (hu2F7 unconjugated antibody) sequences have been disclosed in WO2019154315A1, as detailed below:
The antibody contains the following CDR sequences:

| Name | Sequence | No. |
|---|---|---|
| HCDR1 | GFTFSNYYMS | SEQ ID NO: 1 |
| HCDR2 | YVSSGGGSTYYSDSVKG | SEQ ID NO: 2 |
| HCDR3 | ESYSQGNYFDY | SEQ ID NO: 3 |
| LCDR1 | RASQSISDYLH | SEQ ID NO: 4 |
| LCDR2 | FASQSIS | SEQ ID NO: 5 |
| LCDR3 | QNGHSFSLT | SEQ ID NO: 6 |

hu2F7 HCVR 
EVQLVESGGGLVQPGGSLRLSCAASGFTFSNYYMSWVRQAPGKGLEWVAYVSSGG
GSTYYSDSVKGRFTISRDNAKNTLYLQMSSLRAEDTAVYYCTRESYSQGNYFDYW
GQGTTVTVSS
SEQ ID NO: 7
 hu2F7 LCVR 
EIVMTQSPATLSLSPGERATLSCRASQSISDYLHWYQQKPGQSPRLLIKFASQSISGIPA
RFSGSGSGTDFTLTISSLEPEDFAVYYCQNGHSFSLTFGQGTKLEIK
SEQ ID NO: 8
hu2F7 HC 
EVQLVESGGGLVQPGGSLRLSCAASGFTFSNYYMSWVRQAPGKGLEWVAYVSSGG
GSTYYSDSVKGRFTISRDNAKNTLYLQMSSLRAEDTAVYYCTRESYSQGNYFDYW
GQGTTVTVSSASTKGPSVFPLAPSSKSTSGGTAALGCLVKDYFPEPVTVSWNSGALT
SGVHTFPAVLQSSGLYSLSSVVTVPSSSLGTQTYICNVNHKPSNTKVDKKVEPKSCD
KTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIE
KTISKAKGQPREPQVYTLPPSREEMTKNQVSLTCLVKGFYPSDIAVEWESNGQPENN
YKTTPPVLDSDGSFFLYSKLTVDKSRWQQGNVFSCSVMHEALHNHYTQKSLSLSPGK
SEQ ID NO: 9
hu2F7 LC

EIVMTQSPATLSLSPGERATLSCRASQSISDYLHWYQQKPGQSPRLLIKFASQSISGIPA
RFSGSGSGTDFTLTISSLEPEDFAVYYCQNGHSFSLTFGQGTKLEIKRTVAAPSVFIFPP
SDEQLKSGTASVVCLLNNFYPREAKVQWKVDNALQSGNSQESVTEQDSKDSTYSL
SSTLTLSKADYEKHKVYACEVTHQGLSSPVTKSFNRGEC
SEQ ID NO: 10

2) Anti-B7-H4 antibody-drug conjugate (compound 34, Shanghai Hansoh BioMedical Co., Ltd.): has been disclosed in WO2020244657 and is prepared according to the preparation method of embodiment 9 in WO2020244657. Compound 34 has the following structure:

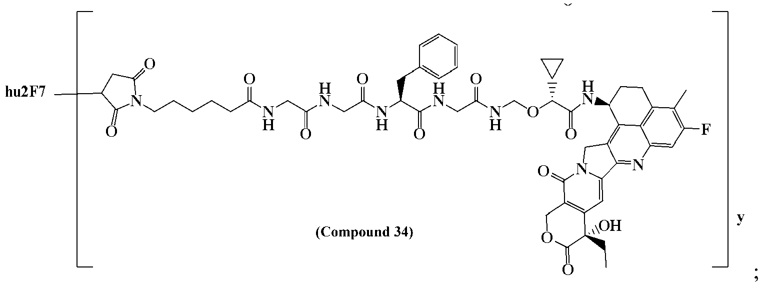

The antibody content C_{mab} is measured by determining the absorbance at 280 nm and the small molecule content C_{Drug} is measured by determining the absorbance at 370 nm, both based on standard calibration curves. Mean drug-to-antibody ratio y = C_{Drug}/C_{mab}. Using the method described above, the mean DAR of the exemplary product hu2F7-exatecan (compound 34) was determined to be 6.1.

### 2.3 Instruments

| Instrument | Model |
|---|---|
| Biosafety cabinet | 1389, Thermo Fisher Scientific |
| Electronic balance | JJ300Y, G&G |
| Electronic balance | BSA124S, Sartorius |
| Analytical balance | CPA225D, Sartorius |
| Vernier calliper | 0-150 mm, Mitutoyo |
| Digimatic calliper | S_Cal EVO Proximity, Sylave |
| Vortex mixer | H-101, Shanghai Kanghe Photoelectric Device Co., Ltd. |
| Pipette | 100-1000 µL, Thermo Fisher Scientific |
| Pipette | 20-200 µL, Thermo Fisher Scientific |
| Pipette | 0.1-10 µL, Thermo Fisher Scientific |

### 2.4 Reagents

| Reagent | Art. No. |
|---|---|
| HBSS | B420KJ, BasalMedia |
| PSB | S120JV, BasalMedia |
| PBS | B320KJ, BasalMedia |

### 3. Experimental Operation and Data Processing

### 3.1 Animals

NU/NU mice, 42-62 days old, purchased from Zhejiang Vitalriver Laboratory Animal Technology Co., Ltd.

### 3.2 Animal modelling

Human cholangiocarcinoma tumour tissue LD1-0060-202214 was evenly cut into about 3 mm x 3 mm x 3 mm (20-30 mg) pieces and subcutaneously implanted into the right flank of NU/NU mice.

### 3.3 Tumour measurement and grouping of tumour-bearing mice

Before the start of treatment, all animals were weighed and their tumour volumes were measured with a vernier calliper as shown below: analyse (mm³) = length (mm) x width (mm) x width (mm)/2. The tumour-bearing mice were randomised based on their weight and tumour volume.

### 3.4 Treatment of tumour-bearing mice

Based on the group, the investigational drug was given (mode of administration: tail vein injection; dose volume:10 mL/kg; dosing frequency: single dose; treatment cycle: 21 days; solvent: PBS solution). After the start of treatment with investigational drug, tumour measurement and weighing were performed twice a week and the animals were euthanised after the end of the experiment.

**Table 2. Administration and Groups**

| **Group** | **Treatment Group** | **N** | **Dose (mg/kg)** | **Dose Regimen** | **Mode of Administration** |
|---|---|---|---|---|---|
| 1 | Vehicle control | 6 | -- | Single dose | *i.v.* |
| 2 | hu2F7 unconjugated antibody | 6 | 10 | Single dose | *i.v.* |
| 3 | Compound 34 | 6 | 10 | Single dose | *i.v.* |

| | | | | | |
|---|---|---|---|---|---|
| *Note: N: number of animals used; i.v.: tail vein injection; vehicle control: PBS solution.* | | | | | |

### 3.5 Data processing

Data were analysed using GraphPad Prism and similar statistical software. The antitumour effect of the compound was assessed using TGI (%) and △T/△C (%). ΔT/ΔC (%) = (T - T0)/(C - C0) x 100, wherein T and C represent the tumour volume in the treatment group and vehicle control group at the end of the experiment respectively, and T0 and C0 are the tumour volume in the treatment group and vehicle control group at the start of the experiment respectively. tumour growth inhibition (TGI) (%) = 100 - ΔT/ΔC (%). Tumour regression was calculated as TGI (%) = 100 - (T - T0)/T0 x 100

### 4. Experimental Results

The tumour growth inhibition effect of anti-B7-H4 antibody-drug conjugate on subcutaneous xenografts of human cholangiocarcinoma LD1-0060-202214 is shown in Table 3.

**Table 3. Pharmacodynamic Parameters**

| **Group** | **Tumour Volume (mm³, Mean ± SEM)** | | **△T/△C (%)** | **TGI (%)** |
|---|---|---|---|---|
| | **Day 0** | **Day 21** | **Day21** | **Day 21** |
| Vehicle control | 122.25 ± 3.97 | 470.26 ± 73.86 | / | / |
| hu2F7 unconjugated antibody 10 mg/kg | 121.91±3.97 | 525.97 ± 71.92 | 116.11 | -16.11 |
| Compound 34 10 mg/kg | 121.89 ± 3.44 | 216.02 ± 38.40 | 27.05 | 72.95 |

| | | | | |
|---|---|---|---|---|
| *Remark: Pharmacodynamic assessment was based on tumour volume measured on Day 21. The data in parentheses indicate the tumour volume of the vehicle group (control group) at that time in the embodiment.* | | | | |

All investigational drugs were administered as a single intravenous dose, and animals were monitored for three consecutive weeks. On the day of the end of experiment (Day 21), the vehicle control group, hu2F7 unconjugated antibody_10 mg/kg dose group and compound 34_10 mg/kg dose group showed a mean tumour volume of 470.26 ± 73.86 mm³, 525.97 ± 71.92 mm³ and 216.02 ± 38.40 mm³ in the tumour-bearing mice respectively. Compared with the vehicle control group, the hu2F7 unconjugated antibody_10 mg/kg dose group and compound 34_10 mg/kg dose group exhibited tumour growth inhibition (TGI) (%) of -16.11% and 72.95% respectively and △T/△C (%) of 116.11% and 27.05% respectively. The statistical data showed a statistically significant decrease (p <0.05) in the mean tumour volume in the compound 34 dose group compared with the vehicle group.

### 5. Experimental Conclusion

In the embodiments of the present invention, the anti-B7-H4 antibody-drug conjugate (ie, compound 34) demonstrated a significant tumour-suppressive effect. Following a single tail vein injection and three weeks of observation, compound 34 significantly inhibited tumour growth in the xenograft model of human cholangiocarcinoma LD1-0060-202214 in nude mice. No significant weight loss or other adverse symptoms related to the treatment were observed.

While the present invention has been described above with reference to various embodiments, it should be understood that the invention should in no way be construed as being limited thereto. The invention encompasses the general principles disclosed herein, and modifications or variations may be made by those skilled in the art without departing from the spirit and scope of the invention. Accordingly, this specification is intended to serve as an illustration, not a limitation.

## Claims

1. The use of an anti-B7-H4 antibody-drug conjugate or its pharmaceutically acceptable salts, stereoisomers, metabolites or solvent compounds in preparation of drugs for prevention and/or treatment of cancers, wherein the anti-B7-H4 antibody-drug conjugate has the structure represented by formula (I): 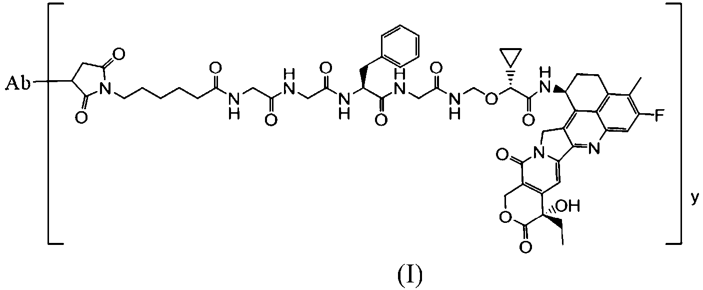
wherein y represents 1 to 20, preferably, y represents 2 to 8, and more preferably, y represents 4 to 6 or 7 to 8; y is a decimal or integer, and further preferably, y represents 5.6, 5.7, 5.8, 5.9, 6.0, 6.1, 6.2, 6.3, or 6.4;
Ab represents an anti-B7-H4 antibody or an antigen-binding fragment thereof, comprising a variable region of antibody heavy chain and a variable region of antibody light chain, wherein the variable region of antibody heavy chain comprises HCDR1, HCDR2 and HCDR3 as shown in SEQ ID NO:1, SEQ ID NO:2 and SEQ ID NO:3 respectively; the variable region of antibody light chain comprises LCDR1, LCDR2 and LCDR3 as shown in SEQ ID NO:4, SEQ ID NO:5 and SEQ ID NO:6 respectively; and
the said cancers are those with low B7-H4 expression.

2. The use according to Claim 1, **characterised in that** the B7-H4 antibody or its antigen-binding fragment is a humanised antibody or its fragment.

3. The use according to Claim 2, **characterised in that** the humanised antibody comprises a variable region of heavy chain as shown in SEQ ID NO:7 and a variable region of light chain as shown in SEQ ID NO:8; preferably, the humanised antibody comprises a heavy chain as shown in SEQ ID NO:9 and a light chain as shown in SEQ ID NO:10.

4. The use according to any of Claims 1-3, **characterised in that** the cancers with low B7-H4 expression where the Immunohistochemistry (IHC) expression level of B7-H4 is 1+ or 2+ B7-H4.

5. The use according to Claim 4, **characterised in that** the cancers with low B7-H4 expression are cholangiocarcinoma with low B7-H4 expression, preferably cholangiocarcinoma with an IHC 1+ or 2+ B7-H4 expression level.

6. The use according to any of Claims 1-5, wherein the prevention and/or treatment includes administration of a therapeutically effective dose of the anti-B7-H4 antibody-drug conjugate in formula (I) or its pharmaceutically acceptable salts, stereoisomers, metabolites or solvent compounds to those in need at 0.5-15 mg/kg of body weight, preferably at 1-10 mg/kg of body weight, and more preferably at 3-10 mg/kg of body weight.

7. The use according to any of Claims 1-5, wherein the prevention and/or treatment includes administration of a therapeutically effective dose of the anti-B7-H4 antibody-drug conjugate in formula (I) or its pharmaceutically acceptable salts, stereoisomers, metabolites or solvent compounds to those in need via oral, parenteral, or transdermal route, wherein the parenteral route is selected from intravenous, subcutaneous or intramuscular injection.

8. The use according to any of Claims 1-5, wherein the prevention and/or treatment includes administration of a therapeutically effective dose of the anti-B7-H4 antibody-drug conjugate in formula (I) or its pharmaceutically acceptable salts, stereoisomers, metabolites or solvent compounds to those in need, wherein the drug is an injectable form, for example, subcutaneous or intravenous injection; the preferable injectable form is injection or lyophilised powder for injection, comprising the anti-B7-H4 antibody-drug conjugate in formula (I) or its pharmaceutically acceptable salts, stereoisomers, metabolites or solvent compounds, and buffers, stabilisers, pH regulators and optional existing surfactants.

9. A method for prevention and/or treatment of cancers with low B7-H4 expression, wherein the method includes administration of a therapeutically effective dose of the anti-B7-H4 antibody-drug conjugate in formula (I) as defined in any of Claims 1-3 or its pharmaceutically acceptable salts, stereoisomers, metabolites or solvent compounds to those in need, preferably at 0.5-15 mg/kg of body weight, and more preferably at 3-10 mg/kg of body weight.
or, the method includes administration of a drug combination with a therapeutically effective dose of the anti-B7-H4 antibody-drug conjugate in formula (I) as defined in any of Claims 1-3 or its pharmaceutically acceptable salts, stereoisomers, metabolites or solvent compounds to those in need.

10. The method for prevention and/or treatment of cancers with low B7-H4 expression according to Claim 9, **characterised in that** the cancers with low B7-H4 expression are those with an IHC 1+ or 2+ B7-H4 expression level; preferably, the cancers with low B7-H4 expression are bile duct cancers with low B7-H4 expression; and more preferably, the cancers with low B7-H4 expression are bile duct cancers with an IHC 1+ or 2+ B7-H4 expression level.

11. The method for prevention and/or treatment of cancers with low B7-H4 expression according to Claim 9, **characterised in that** the anti-B7-H4 antibody-drug conjugate in formula (I) or its pharmaceutically acceptable salts, stereoisomers, metabolites or solvent compounds are administered via oral, parenteral, or transdermal route, wherein the parenteral route is selected from intravenous, subcutaneous or intramuscular injection.

12. The method for prevention and/or treatment of cancers with low B7-H4 expression according to Claim 9, **characterised in that** the anti-B7-H4 antibody-drug conjugate in formula (I) or its pharmaceutically acceptable salts, stereoisomers, metabolites or solvent compounds are injectable forms, for example, subcutaneous or intravenous injection; the more preferable injectable form is injection or lyophilised powder for injection, comprising the anti-B7-H4 antibody-drug conjugate in formula (I) or its pharmaceutically acceptable salts, stereoisomers, metabolites or solvent compounds, and buffers, stabilisers, pH regulators and optional existing surfactants.

13. A drug formulation including a therapeutically effective dose of the anti-B7-H4 antibody-drug conjugate in formula (I) as defined in any of Claims 1-3 or its pharmaceutically acceptable salts or metabolites or solvent compounds, which also includes a pharmaceutically acceptable carrier, wherein the anti-B7-H4 antibody-drug conjugate is administered at 0.5-15 mg/kg of body weight, preferably at 3-10 mg/kg of body weight andis used to treat cancers with low B7-H4 expression; preferably, the cancers with low B7-H4 expression are those with an IHC 1+ or IHC 2+ B7-H4 expression level; more preferably, the cancers with low B7-H4 expression are bile duct cancers with low B7-H4 expression; and further preferably, the cancers with low B7-H4 expression are bile duct cancers with an IHC 1+ or IHC 2+ B7-H4 expression level.
